# EUROPEAN PATENT SPECIFICATION

(11) **EP 1 186 310 B1**
(45) Date of publication and mention of the grant of the patent: **08.11.2006**
(21) Application number: 01121589.4
(22) Date of filing: 10.09.2001
(51) Int. Cl.: A61M 1/10, F04D 13/02

(54) **Turbo blood pump**
Turboblutpumpe
Pompe turbo à sang

(30) Priority: 11.09.2000 JP 2000275436
(43) Date of publication of application: 13.03.2002
(73) Proprietor: JMS Co., Ltd., Hiroshima-shi, Hiroshima 730-8652 (JP)
(72) Inventor: Maeda, Hiroyuki, Hiroshima-shi, Hiroshima 730-8652 (JP); Sato, Masafumi, Hiroshima-shi, Hiroshima 730-8652 (JP); Araki, Kenji, Miyazaki-shi, Miyazaki 880-0943 (JP); Anai, Hirofumi, Oita-shi, Oita 870-0851 (JP)
(74) Representative: Schwarzensteiner, Marie-Luise

(56) References cited:
- EP-A- 0 425 257
- EP-A- 0 504 863
- US-A- 2 810 349
- US-A- 3 558 948
- US-A- 5 863 179

## Description

The present invention relates to a blood pump for transferring blood. In particular, the present invention relates to a turbo blood pump that causes blood to flow by the rotation of an impeller.

Blood pumps are indispensable for conducting extracorporeal circulation by means of artificial heart lung apparatuses or the like. The blood pumps mainly used at present are turbo blood pumps. The turbo blood pump has the following structure: A housing with a pumping chamber formed inside is provided with an inlet port for introducing blood thereinto at its center and an outlet port for discharging blood therefrom on its periphery. An impeller is disposed in the pumping chamber, the rotation of which gives blood a centrifugal force or a driving force in the axial or oblique direction, thereby causing the blood to flow.

To rotate the impeller by a motor, the pump needs a mechanism for transmitting the rotation of the motor disposed outside the housing to the impeller disposed inside the housing. As an example of such transmission mechanism, the structure is known in which the housing is provided with an aperture so that the rotary shaft of the motor penetrates the housing therethrough to be connected to the impeller with a shaft seal separating the inside and outside of the housing. In this case, however, the heat generated at the shaft seal may bring about problems such as damage to blood and the formation of thrombus. In addition, the shaft seal cannot completely separate the inside and outside of the housing, thereby allowing the invasion of bacteria etc.

In order to solve the above problems, the use of magnetic coupling as a means of connecting an impeller and a motor has been devised. The use of the magnetic coupling can eliminate the necessity of the structure in which a drive shaft of the motor for transmitting rotation to the impeller penetrates the wall of the housing.

In the general structure for inducing magnetic coupling, magnets are mounted on the impeller and on the rotor provided outside the housing, and the magnets on the impeller and those on the rotor are arranged in close proximity. When the rotor is rotated by a motor, the impeller rotates following the rotation of the rotor through the magnetic attraction force.

JP 9(1997)-313600 A discloses an example of the turbo blood pump employing the magnetic coupling. In this example, magnets are mounted on the lower surface of the impeller and on the upper surface of a rotor. Accordingly, the magnets on the impeller and those on the rotor face each other in the vertical direction via the wall of the housing, and the rotation of the rotor is transmitted to the impeller through the magnetic attraction force acting in the vertical direction. In the blood pump of this type, since the attraction force caused by the magnets acts vertically, the lower side bearing for rotatably supporting the impeller mainly is exposed to the load. Therefore, an attempt to obtain a sufficiently strong magnetic coupling force increases abrasion of the lower side bearing, which may result in degraded pump performance and an adverse effect on the life of the pump.

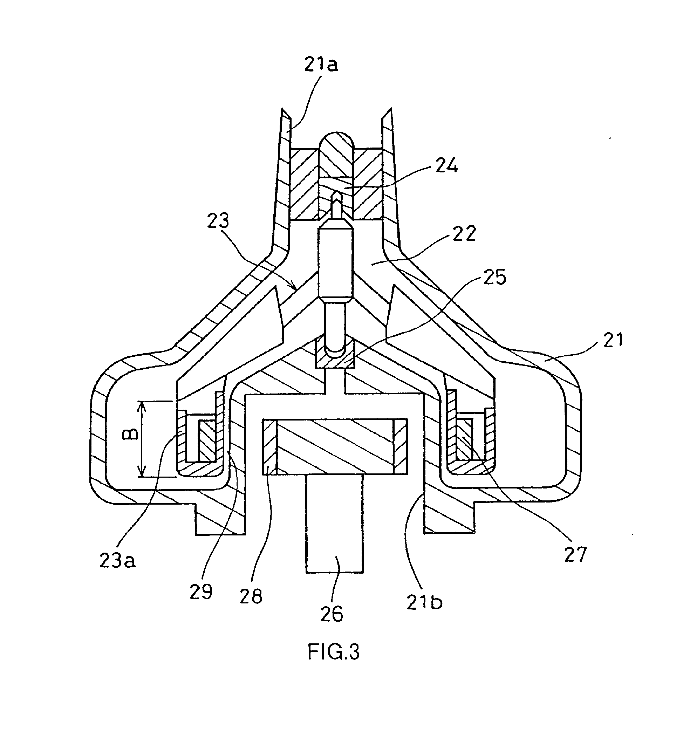

Further, as an another example of magnetic coupling, the inventors of the present invention have studied the structure in which magnets on an impeller and those on a rotor are arranged so as to face each other in the radial direction. FIG. 3 shows an example of this structure. In FIG. 3, reference numeral 21 denotes a housing, which includes an inlet port 21a and an outlet port (not shown in the drawing). In a pumping chamber 22 provided inside the housing 21, an impeller 23 is disposed. The impeller 23 is rotatably supported by an upper bearing 24 and a lower bearing 25. In a recess 21b formed at the center portion of the lower part of the housing 21, a rotor 26 is disposed. Although it is not shown in the drawing, the rotor 26 is rotationally driven by a motor to which it is connected. Driven magnets 27 are mounted on the lower part of the impeller 23 so as to be placed inside the side wall of the recess 21b of the housing 21. On the other hand, driving magnets 28 are mounted on the rotor 26 so as to be placed outside the side wall of the recess 21b. Accordingly, the rotation of the rotor 26 is transmitted to the impeller 23 through the magnetic attraction force acting in the radial direction between the driven magnets 27 and the driving magnets 28.

In the blood pump of this type, to sufficiently increase an area of the portions where the driven magnets 27 and the driving magnets 28 face each other, respectively, for the purpose of obtaining a sufficiently strong magnetic coupling force, both the magnets need to have a sufficient length in the axial direction of the impeller 23. Accordingly, the area B of the outermost peripheral surface 23a of the impeller 23 is increased. Since the outermost peripheral surface 23a has the greatest peripheral velocity, an increase in this area means an increase in the shear stresses on blood, which may increase the chances of blood damage (hemolysis). In addition, since blood stagnation portions 29 are liable to be formed in spaces between the portions on which the driven magnets 27 are mounted and the side wall of the recess 21b, an increase in the volume of these spaces is not preferable because it increases the changes of thrombus formation.

EP 0 425 257 A2 discloses a centrifugal blood pump in which a compact impeller is supported via a pin in a lower bearing of the pump chamber. Driven magnets on the impeller and driving magnets are facing each other via the housing wall in an inclined direction to the rotation axis.

Therefore, with the foregoing in mind, it is an object of the present invention to provide a turbo blood pump that ensures sufficiently strong magnetic coupling between an impeller and a rotor disposed outside a housing, prevents the load concentration to a lower bearing supporting the impeller, and suffers little risk of hemolysis and the formation of blood stagnation portions.

A turbo blood pump according to the present invention is defined in claim 1. A preferred embodiment is given in claim 2.

According to the turbo blood pump of the invention, since the direction of the magnetic coupling inclines, the load applied downward to the lower bearing, which is caused by the magnetic attraction force between the impeller and the rotor, can be reduced. Thus, the magnetic coupling can be made sufficiently strong. Moreover, since the driven magnets and the driving magnets are disposed on oblique surfaces, an area of the portions where the driven magnets and the driving magnets face each other, respectively, can be increased easily without increasing the axial size of the impeller. Therefore, an area of the portions coming into contact with blood at high peripheral velocity is decreased, thereby reducing the chances of hemolysis. In addition, blood stagnation portions also are made smaller, thereby reducing the formation of thrombus.

Further, it is also preferable that the number of the driven magnets and the number of the driving magnets are in a range of 4 to 8, respectively, and the driven magnets and the driving magnets are arranged at substantially uniform intervals on circumferences centered on the rotational axis of the impeller, respectively.

In the turbo blood pump of the invention, the oblique surface in the upper part of the rotor preferably protrudes toward the housing so as to form a convex shape.

These and other advantages of the present invention will become apparent to those skilled in the art upon reading and understanding the following detailed description with reference to the accompanying figures.
FIG. 1 is a cross-sectional view of a turbo blood pump according to an embodiment of the present invention;
FIG. 2A is a plan view of a rotor of the turbo blood pump shown in FIG. 1 and FIG. 2B is a front view of the same; and
FIG. 3 is a cross-sectional view of one example of a turbo blood pump employing magnetic coupling, but not in accordance with the present invention.

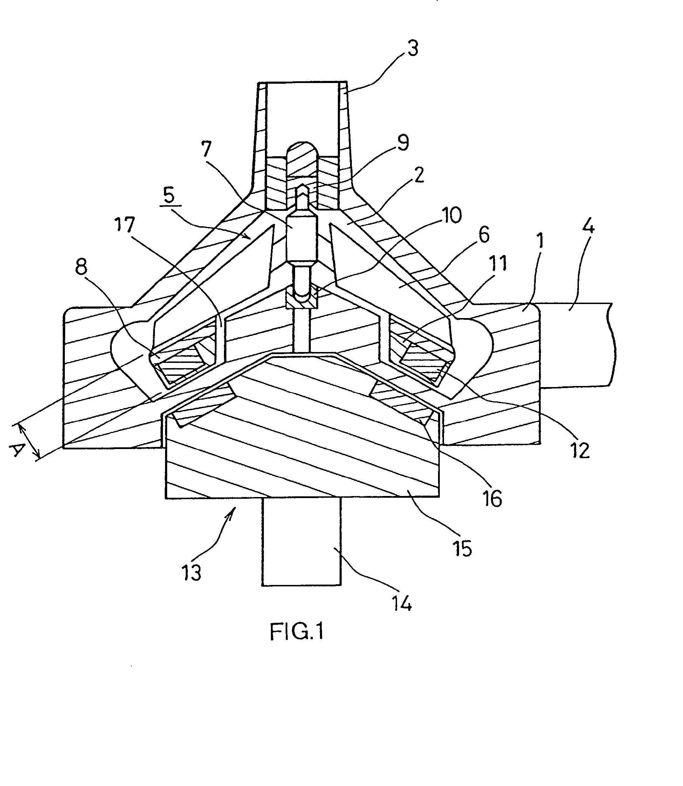

FIG. 1 is a cross-sectional view of a turbo blood pump according to one embodiment of the present invention. In FIG. 1, reference numeral 1 denotes a housing, which includes a pumping chamber 2 for passing blood to cause the blood to flow. The housing 1 is provided with an inlet port 3 communicating with the upper part of the pumping chamber 2 and an outlet port 4 communicating with the side part of the pumping chamber 2. Inside the pumping chamber 2, an impeller 5 is disposed. The impeller 5 includes six vanes 6, a rotary shaft 7, and a ring-shaped annular connecting member 8. The inner portion of each vane 6 is connected to the rotary shaft 7 whereas the outer edge thereof is connected to the annular connecting member 8. The rotary shaft 7 is rotatably supported by an upper bearing 9 and a lower bearing 10, which are both provided in the housing 1. The annular connecting member 8 is provided with magnet cases 11, in which driven magnets 12 may be embedded, respectively. The annular connecting member 8 may have six driven magnets 12 of a cylindrical shape arranged at predetermined intervals in its circumferential direction.

In the lower part of the housing 1, a rotor 13 is disposed. The rotor 13 includes a drive shaft 14 and a magnetic coupling portion 15 of a substantially cylindrical shape, which are connected with each other. Although it is not shown in the drawing, the drive shaft 14 is rotatably supported and is rotationally driven by a rotation drive source such as a motor. Further, although it is not shown in the drawing, the rotor 13 and the housing 1 are held so that they can keep a constant positional relationship. On the upper surface of the magnetic coupling portion 15, driving magnets 16 may be embedded. As shown in a plan view of the rotor 13 illustrated in FIG. 2A, six driving magnets 16 of a cylindrical shape may be arranged at predetermined intervals in the circumferential direction.

The driving magnets 16 are arranged so as to face the driven magnets 12 via the wall of the housing 1. Accordingly, the rotor 13 and the impeller 5 are magnetically coupled with each other. When the rotor 13 is rotated, the impeller 5 is rotationally driven through the magnetic coupling.

As shown in FIG. 1, the lower surface of the annular connecting member 8 on which the driven magnets 12 are mounted is an oblique surface, which is not perpendicular to the rotary shaft 7 but inclines at a predetermined angle. Also, the upper surface of the magnetic coupling portion 15 on which the driving magnets 16 are mounted is an oblique surface. Thus, the driven magnets 12 and the driving magnets 16 induce the magnetic coupling on the surfaces that incline with respect to the rotation axis of the impeller 5.

If the surfaces inducing the magnetic coupling incline as described above, the magnetic attraction force acting between the impeller 5 and the rotor 13 is induced in the direction inclining with respect to the rotation axis of the impeller 5. As a result, the load applied downward to the lower bearing 10 is reduced. Therefore, the lower bearing 10 suffers with less chance of damage to the bearing, whereby a sufficiently strong magnetic coupling thus can be provided easily.

Moreover, as compared with the structure shown in FIG. 3 in which the magnetic attraction force acts in the radial direction, an area of the portions where the driven magnets 12 and the driving magnets 16 face each other, respectively, can be increased without increasing the size of the outer peripheral surface of the annular connecting member 8 in the axial direction because the driven magnets 12 and the driving magnets 16 are arranged in the oblique surfaces. Therefore, the size A of the surface area of the outermost peripheral portions coming into contact with blood at high peripheral velocity is decreased, thereby reducing the chances of hemolysis. In addition, blood stagnation portions 17 formed between the inner peripheral surface of the annular connecting member 8 and the wall of the housing 1 are also made smaller, thereby reducing the formation of thrombus.

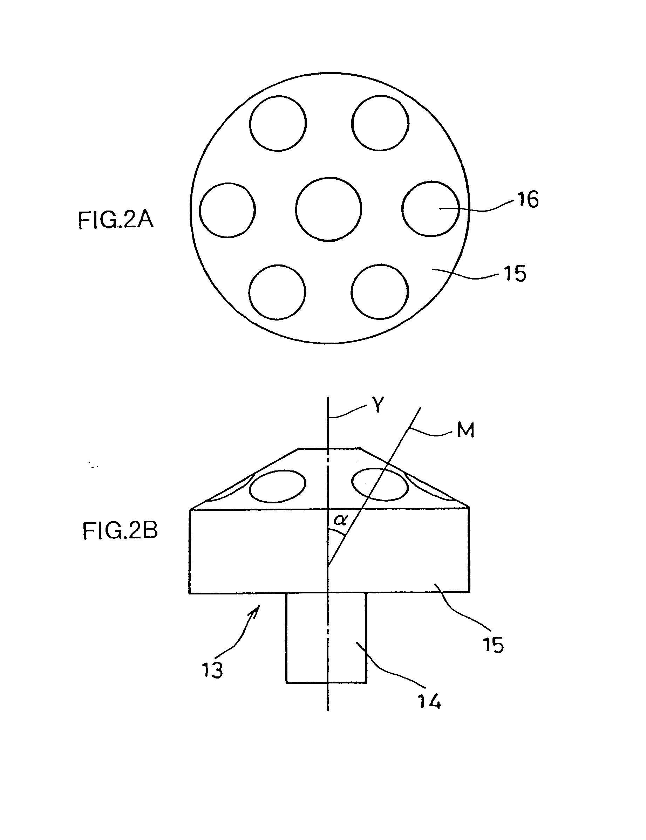

The direction of the magnetic coupling is shown in FIG. 2B by the straight line M perpendicular to the oblique upper surface of the magnetic coupling portion 15. The straight line Y indicates the rotation axis. An angle α at which the direction M of the magnetic coupling inclines with respect to the rotation axis Y is set in the range of 30° ± 15°. When the angle α is in this range, the effects described above can be obtained in a well-balanced state. The angle α greater than this range is not preferable because it can bring about a problem as in the case of the magnetic coupling in the radial direction, i.e., an increase in the surface area of the outer peripheral portions having the high peripheral velocity. Also, the angle α smaller than this range is not preferable because it can bring about a problem as in the case of the magnetic coupling in the vertical direction, i.e., an increase in the load applied to the lower bearing 10.

The oblique surfaces preferably are formed so that the magnetic coupling portion 15 of the rotor 13 protrudes upward to form a convex shape. As compared with the case where the magnetic coupling portion 15 recedes downward to form a concave shape, the space formed between the oblique surfaces and the impeller 5 can be made smaller, which contributes to reducing the size of the pump.

Further, the diameter of the surfaces of the driving magnets 16 facing the driven magnets 12 preferably is greater than that of the surfaces of the driven magnets 12 facing the driving magnets 16. In other words, an area of the surfaces of the driving magnets 16 preferably is greater than that of the surfaces of the driven magnets 12. According to this structure, the torque can be transmitted efficiently through the magnetic coupling.

When more than twelve driven magnets 12 and driving magnets 16 are arranged in the circumferential direction, the spaces between the respective magnets become too close, thereby preventing smooth rotation. Accordingly, it is preferable to arrange four to eight respective magnets in the circumferential direction. When the number of the driven magnets and the driving magnets is both in this range, the pump can provide the most beneficial effect with respect to the driving force and the stability.

The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

## Claims

1. A turbo blood pump, comprising:
a housing (1) having a pump chamber (2), an inlet port (3), and an outlet port (4);
a rotor (13) provided outside the housing (1) and rotationally driven by a motor;
an impeller (5) supported rotatably by a bearing (9,10) in the pump chamber (2) and including a plurality of vanes (6) and a substantially ring-shaped annular connecting member (8) attached to outer parts of the vanes (6);
driven magnets (12) supported by that surface of the annular connecting member (8) that is facing a bottom wall of the housing (1); and
driving magnets (16) mounted on the rotor (13);
the driven magnets (12) and the driving magnets (16) facing each other in parallel via the bottom wall of the housing (1) to form a magnetic coupling, thereby transmitting rotation of the rotor (13) to the impeller (5),
the bottom wall of the housing (1) between the driven magnets and the driving magnets inclines at a predetermined angle with respect to the rotation axis of the impeller (5) and oblique surfaces inclining along the bottom wall are formed in a lower part of the impeller (5) and an upper part of the rotor (13) so that a direction of the magnetic coupling, which is defined based on an attraction force acting between the driven magnets and the driving magnets, inclines with respect to the rotation axis of the impeller (5),
**characterized in that**
the plurality of vanes (6) of the impeller (5) is extending from a rotary shaft (7) that is supported by an upper bearing (9) in the inlet port (3) of the housing (1) and a lower bearing (10) in the center of the bottom wall of the housing (1),
a central area of the bottom wall supporting the lower bearing (10) protrudes upward into the pump chamber (2) with a vertical side surface and subsequent inclined upper surfaces, inclined towards the central axis , the ring-shaped annular connecting member (8) is attached to the underside of the vanes (6) of the impeller (5) and comprises a vertical inner edge parallel and adjacent to the vertical side surface of the central area of the bottom wall, the oblique surface in the upper part of the rotor (13) protrudes toward the housing so as to form a convex shape, and
an angle α at which the direction of the magnetic coupling M inclines with respect to the rotation axis Y of the impeller (5) is set in a range of 30° ± 15°.

2. The turbo blood pump according to claim 1, wherein the number of the driven magnets (12) and the number of the driving magnets (16) are in a range of 4 to 8, respectively, and the driven magnets (12) and the driving magnets (16) are arranged at substantially uniform intervals on circumferences centered on the rotational axis of the impeller (5), respectively.

## Patentansprüche

1. Turboblutpumpe, umfassend:
ein Gehäuse (1) mit einer Pumpenkammer (2), einem Einlassport (3) und einem Auslassport (4);
einen Rotor (13), welcher außerhalb des Gehäuses (1) vorgesehen und rotationsmäßig durch einen Motor angetrieben ist;
ein Flügelrad (5), rotationsmäßig durch ein Lager (9, 10), in der Pumpenkammer (2) unterstützt und umfassend eine Vielzahl von Flügeln (6) und ein im Wesentlichen ringförmiges Verbindungselement (8), das an den äußeren Teilen der Flügel (6) angebracht ist;
angetriebene Magnete (12), welche durch jene Oberfläche des ringförmigen Verbindungselementes (8) getragen werden, welche an eine Bodenwand des Gehäuses (1) angrenzt, und
Antriebsmagnete (16), welche an dem Rotor (13) befestigt sind;
wobei die angetriebenen Magnete (12) und die Antriebsmagnete (16) zueinander parallel über die Bodenwand des Gehäuses (1) unter Ausbildung magnetischer Kopplung, gerichtet sind, wodurch Rotation des Rotors (13) auf das Flügelrad (5) übertragen wird,
sich die Bodenwand des Gehäuses (1) zwischen den angetriebenen Magneten und den Antriebsmagneten unter einem vorbestimmten Winkel in Bezug auf die Rotationsachse des Flügelrads (5) neigt und schiefe Oberflächen, welche entlang der Bodenwand geneigt verlaufen, in einem unteren Teil des Flügelrades (5) und einem oberen Teil des Rotors (13) ausgebildet sind, so dass die Richtung der magnetischen Kopplung, welche basierend auf Anziehungskräften, welche zwischen den angetriebenen Magneten und den Antriebsmagneten wirken, definiert ist, sich in Bezug auf die Rotationsachse des Flügelrades (5) neigt,
**dadurch gekennzeichnet, dass**
eine Vielzahl von Flügeln (6) des Flügelrades (5) sich von einem Rotationsschaft (7) erstrecken, welcher durch ein oberes Lager (9) in dem Einlassport (3) des Gehäuses (1) und ein unteres Lager (10) in der Mitte der Bodenwand des Gehäuses getragen wird,
wobei ein mittiger Bereich der Bodenwand, welcher das untere Lager (10) trägt, nach oben in die Pumpenkammer (2) ragt, mit einer senkrechten Seitenoberfläche und nachfolgenden geneigten oberen Oberflächen, welche gegen die Mittelachse geneigt sind, das ringförmige Verbindungselement (8) an der Unterseite der Flügel (6) des Flügelrades (5) befestigt ist und eine senkrechte Innenkante parallel zu und angrenzend an die senkrechte Seitenoberfläche des mittigen Bereichs der Bodenwand umfasst, die schräge Oberfläche am oberen Teil des Rotors (13) in das Gehäuse unter Ausbildung einer konvexen Form ragt; und
ein Winkel α, bei dem die Richtung der magnetischen Kopplung M in Bezug auf die Rotationsachse Y des Flügelrades (5) geneigt ist, innerhalb eines Bereiches von 30° ± 15° eingestellt ist.

2. Turboblutpumpe gemäß Anspruch 1, worin die Anzahl der angetriebenen Magnete (12) und die Anzahl der Antriebsmagnete (16) jeweils im Bereich von 4 bis 8 liegt und die angetriebenen Magnete (12) und die Antriebsmagnete (16) in im Wesentlichen gleichförmigen Intervallen an Umfängen angeordnet sind, die jeweils an der Rotationsachse des Flügelrades (5) zentriert sind.

## Revendications

1. Pompe-turbine pour du sang, comprenant :
un boîtier (1) ayant une chambre de pompe (2), un orifice d'entrée (3), et un orifice de sortie (4) ;
un rotor (13) prévu à l'extérieur du boîtier (1) et entraîné en rotation par un moteur ;
une hélice (5) supportée en rotation par un palier (9, 10) dans la chambre de pompe (2) et incluant une pluralité d'aubes (6) et un élément de connexion annulaire sensiblement en forme de bague (8) attaché aux parties extérieures des aubes (6) ;
des aimants entraînés (12) supportés par cette surface de l'élément de connexion annulaire (8) qui fait face vers une paroi de fond du boîtier (1) ; et
des aimants d'entraînement (16) montés sur le rotor (13) ;
les aimants entraînés (12) et les aimants d'entraînement (16) se faisant mutuellement face en parallèle via la paroi de fond du boîtier (1) pour former un accouplement magnétique, transmettant ainsi la rotation du rotor (13) à l'hélice (5),
la paroi de fond du boîtier (1) entre les aimants entraînés et les aimants d'entraînement est inclinée sous un angle prédéterminé par rapport à l'axe de rotation de l'hélice (5) et des surfaces obliques inclinées le long de la paroi de fond sont formées dans une partie inférieure de l'hélice (5) et une partie supérieure du rotor (13) de sorte qu'une direction de l'accouplement magnétique, qui est définie en se basant sur une force d'attraction agissant entre les aimants entraînés et les aimants d'entraînement, est inclinée par rapport à l'axe de rotation de l'hélice (5),
**caractérisée en ce que**
la pluralité d'aubes (6) de l'hélice (5) s'étendent depuis un arbre rotatif (7) qui est supporté par un palier supérieur (9) dans l'orifice d'entrée (3) du boîtier (1) et un palier inférieur (10) dans le centre de la paroi de fond du boîtier (1),
une zone centrale de la paroi de fond supportant le palier inférieur (10) se projette vers le haut jusque dans la chambre de pompe (2) avec une surface latérale verticale et avec des surfaces supérieures inclinées ultérieures, qui sont inclinées vers l'axe central, l'élément de connexion annulaire en forme de bague (8) est attaché sur la face inférieure des aubes (6) de l'hélice (5) et comprend un bord intérieur vertical parallèle et adjacent à la surface latérale verticale de la zone centrale de la paroi de fond, la surface oblique dans la partie supérieure du rotor (13) étant en projection vers le boîtier de manière à former une forme convexe et, et
un angle α sous lequel la direction de l'accouplement magnétique M s'incline par rapport à l'axe de rotation Y de l'hélice (5) est choisi dans une plage de 30° +/- 15°.

2. Pompe-turbine pour du sang selon la revendication 1, dans laquelle le nombre des aimants entraînés (12) et le nombre des aimants d'entraînement (16) sont respectivement dans une plage de 4 à 8, et les aimants entraînés (12) et les aimants d'entraînement (16) sont agencés à intervalles sensiblement uniformes sur des circonférences centrées sur l'axe de rotation de l'hélice (5), respectivement.
